# EUROPEAN PATENT SPECIFICATION

(11) **EP 4 311 516 B1**
(45) Date of publication and mention of the grant of the patent: **10.09.2025**
(21) Application number: 22187241.9
(22) Date of filing: 27.07.2022
(51) Int. Cl.: A61C 1/14, A61B 17/16

(54) **DENTAL HANDPIECE HAVING A CHUCK WITH AN INTEGRATED PLUNGER BALL**
ZAHNÄRZTLICHES HANDSTÜCK MIT EINEM SPANNFUTTER MIT INTEGRIERTER KOLBENKUGEL
PIÈCE À MAIN DENTAIRE DOTÉE D'UN MANDRIN INTÉGRANT UNE BALLE DE PISTON

(43) Date of publication of application: 31.01.2024
(73) Proprietor: Dentsply Sirona Inc., York, PA 17401 (US); Sirona Dental Systems GmbH, 64625 Bensheim (DE)
(72) Inventor: Ertugrul, Metin, 64625 Bensheim (DE)
(74) Representative: Allen, Caroline Margaret

(56) References cited:
- EP-A2- 0 245 692
- JP-U- H02 130 619

## Description

### TECHNICAL FIELD OF THE INVENTION

The present invention relates to dental handpieces, in particular to the clamping mechanism of a dental handpiece for releasably clamping a dental drill.

### BACKGROUND ART OF THE INVENTION

A dental handpiece is a handheld device for performing a variety of dental treatments which may require the use of various dental tools such as drills. The head of the dental handpiece is usually provided with a clamping mechanism such as a chuck that allows the dentist to change the dental tool in accordance with the intended dental treatment by means of a push button.

Reference is made to EP0639354A2, EP 1733696A1, EP2959860A1, JPH02130619U, EP0245692A2, each disclosing a prior art dental handpiece.

JP H02 130619 U discloses a dental handpiece comprising: a head;a rotatable hollow shaft which is rotatably arranged inside the head;a driving means for rotating the hollow shaft;a chuck having resilient arms for releasably clamping a dental drill, wherein the chuck is fixed into the hollow shaft so as to rotate with same; a wedge-shaped element which is axially movably arranged inside the rotatable hollow shaft and in direct contact with the resilient arms of the chuck, wherein the wedge-shaped element partly protrudes through an opening on the upper end of the hollow shaft, and wherein the opening is smaller than the size of the wedge-shaped element ; a push button which is arranged in the head and configured to contact, when pressed by a user, the partly protruding wedge-shaped element and move the wedge-shaped element downwardly in between the resilient arms so as flex the resilient arms radially outwards and release the dental drill; and a biasing means to move the push button away from the ball, wherein the push button is not in contact with the wedge-shaped element when it is released.

There are certain design requirements that must be met by the dental handpiece to assure its proper operation. For example, any excessive heating of the push button at the contact area with the clamping mechanism must be prevented in as much as possible. For example, any excessive mechanical abrasion of the clamping jaws must be prevented in as much as possible. For example, the size of the head should be reduced as much as possible so that it can easily reach spatially limited places in the oral cavity. For example, the push button and the clamping mechanism must be operable in a reliable and easy manner.

### DISCLOSURE OF THE INVENTION

The objective of the present invention is to overcome the disadvantages of the prior art and provide a dental handpiece which is mechanically compact and easy to use.

This objective has been achieved through the dental handpiece as defined in claim 1. The subject-matter of the dependent claim relates to a further development and embodiment. The dental handpiece of the present invention comprises: a head; a rotatable hollow shaft which is rotatably arranged inside the head; a driving means for rotating the hollow shaft; a chuck having resilient arms for releasably clamping a dental drill, wherein the chuck is fixed into the hollow shaft so as to rotate with same; a ball which is axially movably arranged inside the rotatable hollow shaft and in direct contact with the resilient arms of the chuck, wherein the ball partly protrudes through an opening on the upper end of the hollow shaft, wherein the opening is smaller than the size of the ball; a push button which is arranged in the head and configured to contact when pressed by a user, the partly protruding ball and move the ball downwardly in between the resilient arms so as flex the resilient arms radially outwards and release the dental drill, wherein the resilient arms bias the ball inside the rotatable hollow shaft towards the opening; and a biasing means adapted to move the push button away from the ball, wherein the push button is not in contact with the ball when it is released.

A major advantageous effect of the present invention is that the chuck can be prevented from any excessive mechanical abrasion. This can be explained as follows: Since the ball rotates together with the hollow shaft, it has *no* relative speed with respect to the resilient arms of the chuck when the user presses the push button. This reduces the friction on the chuck as well as the heat dissipation during pressing operation. Thereby excessive mechanical abrasion and heating of the chuck can be prevented in as much as possible. Meanwhile, the heat generated due to the relative motion of the ball with respect to the push button when the push button contacts the upper side of the ball in a point-like manner will be generally dissipated by the push button in the radial directions. Furthermore, the lower side of the ball generally gives only rise to point-like contacts with the resilient arms of the chuck due to its spherical shape which further reduces any heat transfer from the lower side of the ball to the chuck.

Another major advantageous effect of the present invention is that the size of the head can be further reduced. This can be explained as follows. As the ball is located immediately underside the push button it can be directly used to flex the resilient arms of the chuck upon the downwards movement of the push button for releasing the dental drill, thus the number of parts needed to clamp/release the dental drill has been reduced. This allows a compact mechanical configuration. The compact size of the head will also allow the dentist to easily reach spatially limited places in the oral cavity and to work more easily inside the oral cavity.

Another major advantageous effect of the present invention is that the push button is operable in a more reliable and easy manner. This can be explained as follows. As the ball is located immediately between the push button and the chuck to operate like a plunger, it will directly actuate the release operation. Moreover, when the ball is forced downwards, the contact forces arising between the chuck and the ball will increase gradually in the radially horizontal directions and push the resilient arms away with a gradually increasing magnitude due to the spherical curvature of the ball plunging between the resilient arms. Thereby, the user can apply a comparatively less force for clamping/releasing the dental drill. This allows a comfortable feeling for the user's finger when pressing the button.

In an embodiment, the ball is preferably made of ceramic material. Thereby the friction can be reduced, and mechanical strength can be increased.

### BRIEF DESCRIPTION OF THE DRAWINGS

In the subsequent description, the present invention will be described in more detail by using exemplary embodiments and by referring to the drawings, wherein
Fig. 1 - is a schematic partial side view of a dental handpiece according to an embodiment of the invention;
Fig. 2a - is a schematic partial vertical cross-sectional view of the head of the dental handpiece in Fig 1, taken along the line I-I;
Fig. 2b - is a schematic partial cross-sectional view of the head of the dental handpiece in

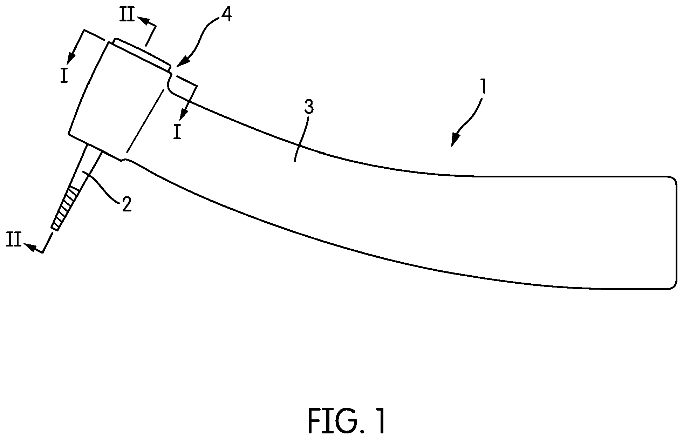

Fig 1, taken along the line I-I in the actuated state;

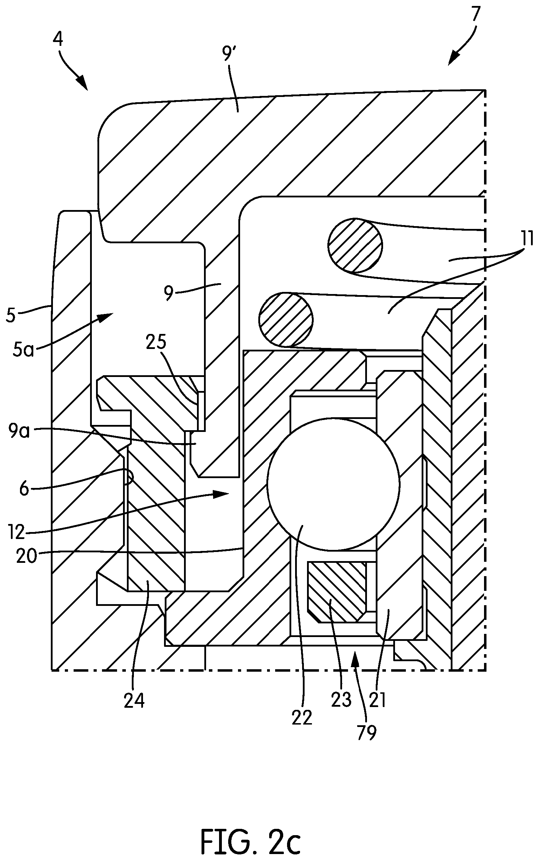

Fig. 2c - is a schematic partial cross-sectional view of the head of a dental handpiece according to an alternative embodiment of the invention, taken along the line II-II in Fig 1;

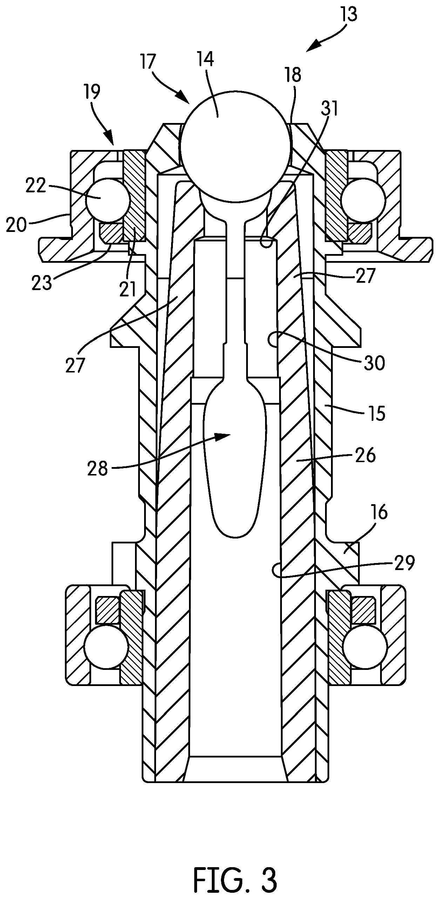

Fig. 3 - is a schematic partial cross-sectional view of the clamping mechanism inside the bearings of the dental handpiece in Fig 1, taken along the line I-I;

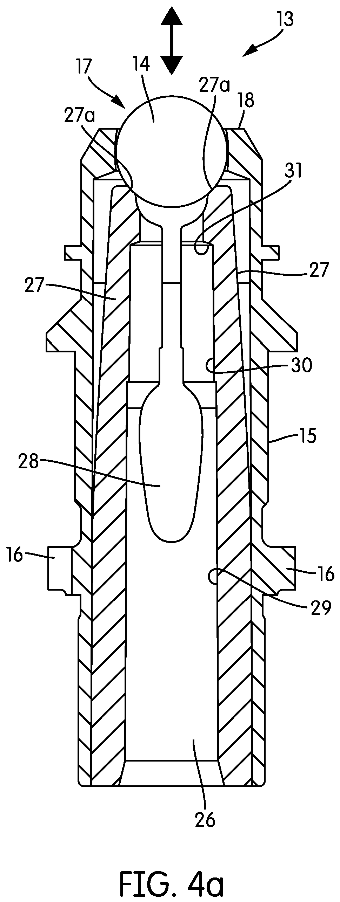

Fig. 4a - is a schematic partial cross-sectional view of the clamping mechanism of Fig. 3 without the bearings;

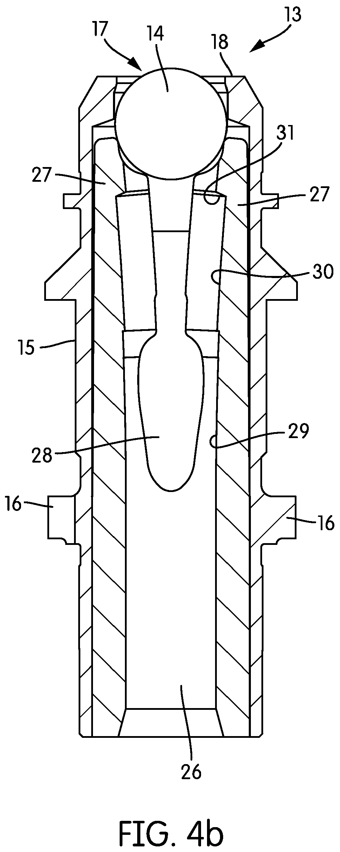

Fig. 4b - is a schematic partial cross-sectional view of the clamping mechanism of Fig 4a, in the actuated state;

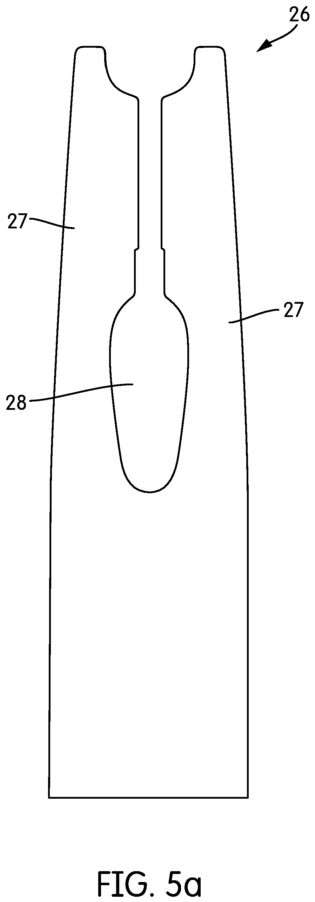

Fig. 5a - is a schematic perspective side view of the chuck of the dental handpiece in Fig 1;

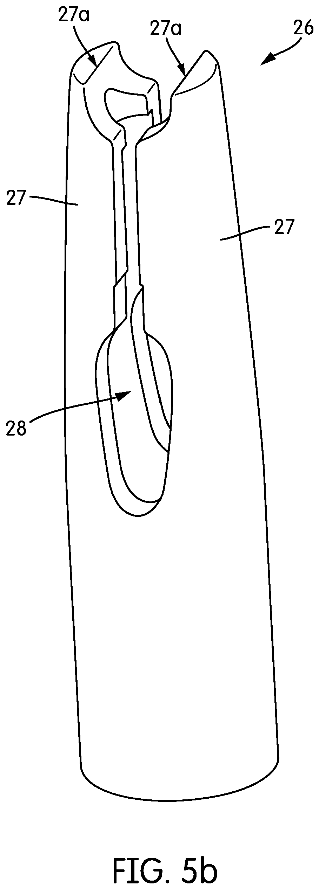

Fig. 5b - is a schematic perspective view of the chuck of the dental handpiece in Fig 1;

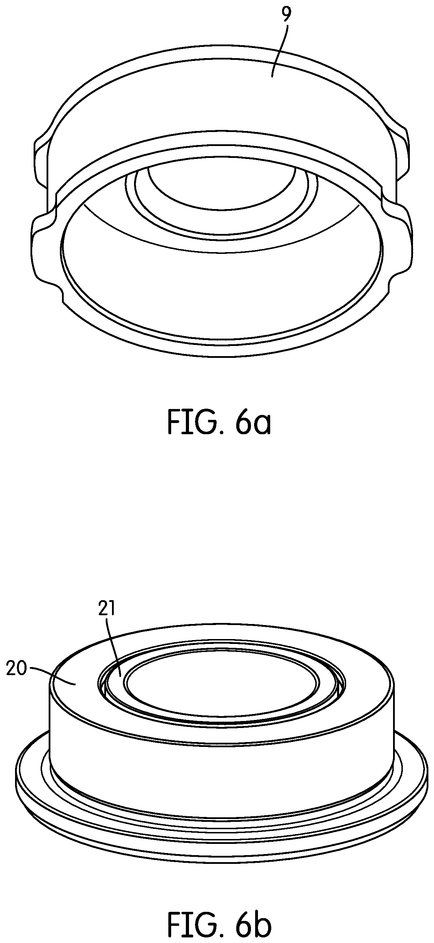

Fig. 6a - is a schematic perspective view of the flange collar (hat-shaped) of the dental handpiece in Fig 1;
Fig. 6b - is a schematic perspective view of the outer and inner races of the dental handpiece in Fig 1;

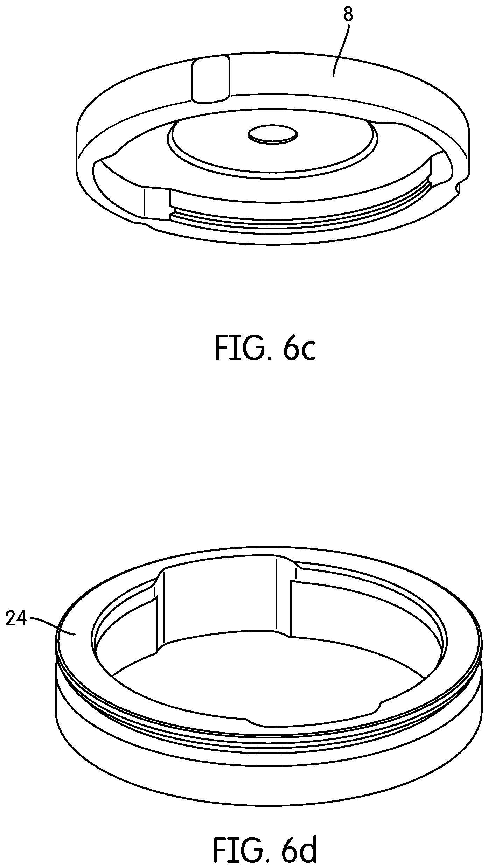

Fig. 6c - is a schematic perspective view of the cap of the dental handpiece in Fig 1;
Fig. 6d - is a schematic perspective view of the screw ring of the dental handpiece in Fig 1.

The reference numbers shown in the drawings denote the elements as listed below and will be referred to in the subsequent description of the exemplary embodiments.
- 1.: Dental handpiece
- 2.: Dental drill (burr)
- 3.: Body
- 4.: Head
- 5.: Housing
- 5a.: Gap
- 6.: Internal Thread
- 7.: Pushbutton
- 8.: Cap
- 9.: Flange collar (hat-shaped)
- 9a.: Claw
- 9b.: Air Gap (thermal insulation)
- 9': Cap
- 10.: Contact surface (point like)
- 11.: Spring (cone shaped)
- 12.: Guide
- 13.: Clamping means
- 14.: Ball
- 15.: Hollow shaft
- 16.: Gear
- 17.: Opening
- 18.: Stop Edge
- 19.: Bearing
- 20.: Outer race
- 21.: Inner race
- 22.: Balls
- 23.: Retainer
- 24.: Screw ring
- 25.: Nose
- 26.: Chuck
- 27.: Arm
- 27a.: Contact point
- 28.: Cut-out
- 29.: Large diameter portion
- 30.: Small diameter portion
- 31.: Contact edge (Burr stop)
- 32.: Driving shaft
- 33.: Bearing
- 34.: Outer race
- 35.: Inner race
- 36.: Balls
- 37.: Retainer
- 38.: Crown gear

### A: Axial Direction

### F: Actuation Force

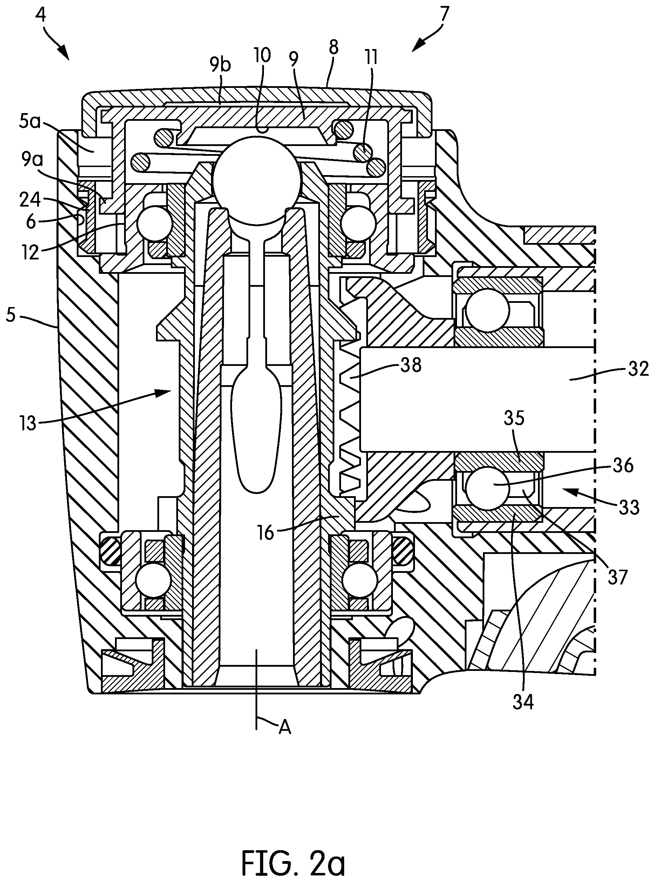

Fig. 1 shows a dental handpiece (1) according to an embodiment of the invention. As shown in Fig. 1, dental handpiece (1) has a body (3) and a head (4). A dental drill (2) is detachably attached to the head (4). Fig. 2a shows an enlarged cross-sectional view of the head (4). As shown in Fig. 2a, the head (4) has a housing (5) which accommodates a clamping means (13). As shown in Fig. 4a the clamping means (13) has a chuck (26) with resilient arms (27) for releasably clamping the dental drill (2). A hollow shaft (15) is rotatably arranged inside the head (4). The chuck (26) is fixed into the hollow shaft (15) to rotate with same. The dental handpiece (1) has a driving means such as a motor (not shown) for rotating the hollow shaft (15). The driving means is coupled to a driving shaft (32) as shown in Fig. 2a, that is rotatably held through a bearing (33) having an outer race (34), an inner race (35), multiple balls (36), and a retainer (37). The end of the driving shaft (32) has a crown gear (38) that meshes with a gear (16) on the hollow shaft (15). As shown in Fig. 3 the hollow shaft (15) is rotatably held through a couple of bearings (19) mounted into the housing (5). The bearing (19) has an outer race (20) and inner race (21), multiple balls (22) and a retainer (23).

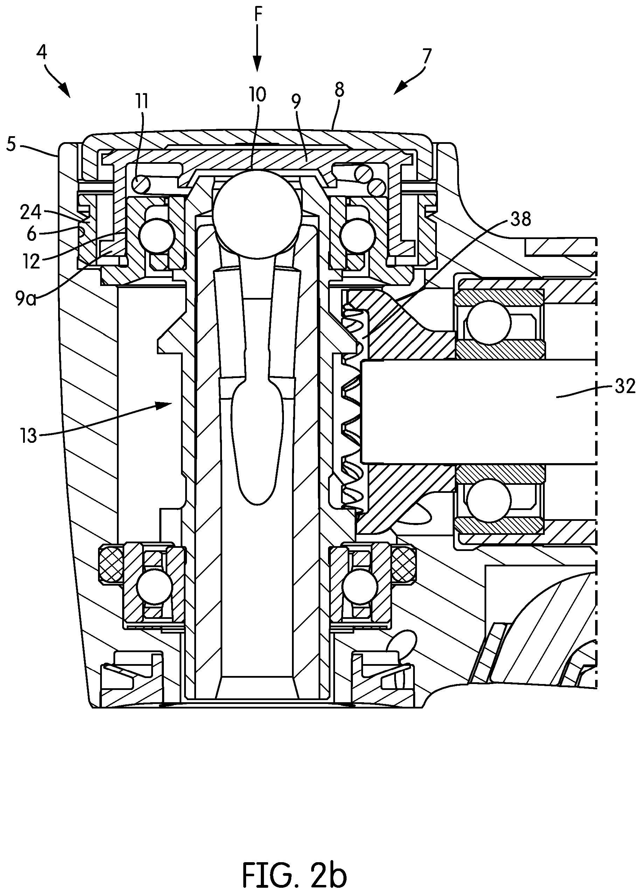

As shown in Fig. 3, the clamping means (13) has a ball (14), preferably made of ceramic material, which is axially movably arranged inside the rotatable hollow shaft (15) and in direct contact with the resilient arms (27) of the chuck (26). The ball (14) partly protrudes through an opening (17) in the upper end of the hollow shaft (15). The size of the opening (17) is smaller than the size of the ball (14) so that it is retained partly within the hollow shaft (15). As shown in Fig. 3 the opening (17) has an annular stop edge (18) which restricts the outwards movement of that ball (14). As shown in Fig. 2a and Fig. 2b the dental handpiece (1) has a push button (7) which is arranged in the head (4) and configured to contact, when pressed by a user, the partly protruding ball (14) and move the ball (14) downwardly in between the resilient arms (27) to flex the resilient arms (27) radially outwards and release the dental drill (2). As shown in Fig. 5a the chuck (26) has two diametrically opposed cut-outs (28) in its upper portion to form the two resilient arms (27). As shown in Fig. 5b, a linear semi cylindrical channel is formed into the upper portion of the chuck (26) whose edges form two diametrically opposed contact points (27a) to contact the ball (14). As shown in Fig. 3, the chuck (26) has a large diameter portion (29) and a small diameter portion (30). When the resilient arms (27) are retracted the dental drill (2) can be manually inserted only until the upper end of the large diameter portion (29). When the resilient arms (27) are flexed, the dental drill (2) can be further inserted into the small diameter portion (30) until it abuts against the contact edge (31) (Burr stop). Once the push button (7) is released, the resilient arms (27) are retracted, and the dental drill (2) is clamped in the small diameter portion (30) between the resilient arms (27). The resilient arms (27) also bias the ball (14) inside the rotatable hollow shaft (15) towards the opening (17). As shown in Fig. 2a, the pushbutton (7) has a cap (8), and a hat-shaped flange collar (9) arranged inside the cap (8) and fixed thereto. The hat-shaped flange collar (9) has a point like contact surface (10), preferably planar, for the ball (14). The pushbutton (7) has a biasing means such a spring (11), preferably a cone shaped spring (11) to move the push button (7) away and detach it from the ball (14). As shown in Fig. 2a, the point like contact surface (10) is not in contact with the ball (15) when the push button (7) is released. As shown in Fig. 2b, the contact surface (10) comes in point contact with the upper side of the ball (15) when the push button (7) is pressed downwards.

Fig. 2c shows an alternative embodiment, wherein the cap (9') is provided as a single piece instead of two separate pieces. That means, the cap (8) and the hat-shaped Flange collar (9) of Fig. 2a can also be provided in the form of a single piece with a similar function. All other features remain the same. Thereby the number of pieces can be further reduced.

As shown in Fig. 2a, the housing (5) has an internal thread (6) which receives a screw ring (24). The screw ring (24) has a nose (25) which can be seen in Fig. 2c but not in Fig.2a. The cross-sectional views respectively in Fig. 2a and Fig. 2c are rotated about 90 degrees with respect to each other. The cross-section in Fig. 2a is along the line I-I as shown in Fig. 1 and the cross section in Fig. 2c is along the line II-II as shown in Fig. 1. As shown in Fig. 2c, the nose (25) extends radially inwards and can abut from below against the claw (9a) of the hat-shaped flange collar (9) which extends radially outwards and prevents the push button (7) from disengaging from the housing (5). The hat-shaped flange collar (9) can be provided separately from or integrally with the cap (8;9'). In the former case an air gap (9b) is preferably formed underneath the cap (8) to improve thermal insulation as shown in Fig. 2a. Alternatively the air gap (9b) may be filled with a material that is a poor heat-conductor.

As shown in Fig. 2a and Fig.2c, the inner surface of the hat-shaped flange collar (9) slides on the outer surface of outer race (20) while being in contact with the latter which configures a guide (12) for the push button (7) movement. The nose (25) of the screw ring (24) is arranged above the claw (9a) of the hat-shaped flange collar (9). The hat-shaped flange collar (9) does not contact the inner radial surface of the screw ring (24). The housing (5) has a gap (5a) directly above the screw ring (24) and the nose (25), which allows the cap (8;9) to move upwards or downwards during the clamping/releasing operation. The outer cylindrical surface of the cap (8;9') does not contact the inner surface of the housing (5) during its entire movement thanks to a sufficient radial clearance. Thus, guide (12) serves as a guiding contact surface for the up/down movement of the cap (8;9).

Through the above described configuration, the height and diameter of the head (4) can be further reduced.

## Claims

1. A dental handpiece (1) comprising:
a head (4);
a rotatable hollow shaft (15) which is rotatably arranged inside the head (4);
a driving means for rotating the hollow shaft (15);
a chuck (26) having resilient arms (27) for releasably clamping a dental drill (2),
wherein the chuck (26) is fixed into the hollow shaft (15) so as to rotate with same;
a ball (14) which is axially movably arranged inside the rotatable hollow shaft (15) and in direct contact with the resilient arms (27) of the chuck (26), wherein the ball (14) partly protrudes through an opening (17) on the upper end of the hollow shaft (15), and
wherein the opening (17) is smaller than the size of the ball (14);
a push button (7) which is arranged in the head (4) and configured to contact, when pressed by a user, the partly protruding ball (14) and move the ball (14) downwardly in between the resilient arms (27) so as flex the resilient arms (27) radially outwards and
release the dental drill (2), wherein the resilient arms (27) bias the ball (14) inside the rotatable hollow shaft (15) towards the opening (17); and
a biasing means to move the push button (7) away from the ball (14), wherein the push button (7) is not in contact with the ball (15) when it is released.

2. The dental handpiece (1) according to claim 1, **characterized in that** the ball (14) is made of ceramic material.

## Patentansprüche

1. Zahnärztliches Handstück (1), umfassend:
einen Kopf (4);
eine drehbare Hohlwelle (15), die drehbar innerhalb des Kopfes (4) angeordnet ist;
ein Antriebsmittel zum Drehen der Hohlwelle (15);
ein Spannfutter (26) mit elastischen Armen (27) zum freigebbaren Festklemmen eines zahnärztlichen Bohrers (2), wobei das Spannfutter (26) in der Hohlwelle (15) befestigt ist, um sich mit dieser zu drehen;
eine Kugel (14), die axial bewegbar innerhalb der drehbaren Hohlwelle (15) angeordnet ist und in direktem Kontakt mit den elastischen Armen (27) des Spannfutters (26) steht, wobei die Kugel (14) teilweise durch eine Öffnung (17) an dem oberen Ende der Hohlwelle (15) vorsteht und wobei die Öffnung (17) kleiner als die Größe der Kugel (14) ist;
einen Druckknopf (7), der in dem Kopf (4) angeordnet und dazu konfiguriert ist, wenn er von einem Benutzer gedrückt wird, die teilweise vorstehende Kugel (14) zu berühren und die Kugel (14) zwischen den elastischen Armen (27) nach unten zu bewegen, um die elastischen Arme (27) radial nach außen zu biegen und den zahnärztlichen Bohrer (2) freizugeben, wobei die elastischen Arme (27) die Kugel (14) innerhalb der drehbaren Hohlwelle (15) in Richtung der Öffnung (17) vorspannen; und
ein Vorspannmittel, um den Druckknopf (7) von der Kugel (14) weg zu bewegen, wobei der Druckknopf (7) nicht in Kontakt mit der Kugel (15) steht, wenn er freigegeben wird.

2. Zahnärztliches Handstück (1) nach Anspruch 1, **dadurch gekennzeichnet, dass** die Kugel (14) aus keramischem Material hergestellt ist.

## Revendications

1. Pièce à main dentaire (1) comprenant :
une tête (4) ;
un arbre creux (15) rotatif qui est agencé de manière rotative à l'intérieur de la tête (4) ;
un moyen d'entraînement destiné à faire tourner l'arbre creux (15) ;
un mandrin (26) doté de bras élastiques (27) destiné à serrer de manière libérable un foret dentaire (2), dans laquelle le mandrin (26) est fixé dans l'arbre creux (15) de façon à tourner avec celui-ci ;
une balle (14) qui est agencée de manière axialement mobile à l'intérieur de l'arbre creux (15) rotatif et en contact direct avec les bras élastiques (27) du mandrin (26), dans laquelle la balle (14) fait partiellement saillie à travers une ouverture (17) sur l'extrémité supérieure de l'arbre creux (15), et dans laquelle l'ouverture (17) est plus petite que la taille de la balle (14) ;
un bouton poussoir (7) qui est agencé dans la tête (4) et conçu pour entrer en contact, lorsqu'il est pressé par un utilisateur, avec la balle partiellement saillante (14) et déplacer la balle (14) vers le bas entre les bras élastiques (27) de façon à fléchir les bras élastiques (27) radialement vers l'extérieur et libérer le foret dentaire (2), dans laquelle les bras élastiques (27) sollicitent la balle (14) à l'intérieur de l'arbre creux (15) rotatif vers l'ouverture (17) ; et
un moyen de sollicitation pour éloigner le bouton poussoir (7) de la balle (14), dans laquelle le bouton poussoir (7) n'est pas en contact avec la balle (15) lorsqu'il est libéré.

2. Pièce à main dentaire (1) selon la revendication 1, **caractérisée en ce que** la balle (14) est constituée d'un matériau céramique.
